# EUROPEAN PATENT SPECIFICATION

(11) **EP 2 763 711 B1**
(45) Date of publication and mention of the grant of the patent: **20.03.2019**
(21) Application number: 12783432.3
(22) Date of filing: 05.10.2012
(51) Int. Cl.: A61L 31/02, A61L 31/14

(54) **MAGNESIUM ALLOYS FOR BIOABSORBABLE STENT**
MAGNESIUMLEGIERUNGEN FÜR EINEN BIOABSORBIERBAREN STENT
ALLIAGES DE MAGNÉSIUM POUR ENDOPROTHÈSE BIOABSORBABLE

(30) Priority: 07.10.2011 US 201161544373 P
(43) Date of publication of application: 13.08.2014
(73) Proprietor: Medtronic Vascular, Inc., Santa Rosa, CA 95403 (US)
(72) Inventor: GUO, Ya, Santa Rosa California 95403 (US); MISRA, Abhijeet, Santa Rosa California 95403 (US); WRIGHT, James A., Santa Rosa California 95403 (US); BERGLUND, Joseph, Santa Rosa California 95403 (US); PRASANNAVENKATESAN, Rajesh, Santa Rosa California 95403 (US)
(74) Representative: Zimmermann & Partner Patentanwälte mbB
(86) International application number: PCT/US2012/059038
(87) International publication number: WO 2013/052856

(56) References cited:
- EP-A1- 2 169 090
- WO-A1-2011/051424
- US-A1- 2009 306 765

## Description

### FIELD

The present invention is related to magnesium-based alloys that may be used to manufacture bioabsorbable stents.

### BACKGROUND

Magnesium alloys demonstrate excellent specific properties that make them potentially suitable candidates for replacing heavier materials in several commercial, defense, and medical applications. Magnesium is less toxic and an attractive alloy for biodegradable medical implant applications. Potential limitations of the current magnesium alloys include low ductility, low strength, limited high-temperature properties and poor corrosion resistance. Magnesium has a hexagonally close-packed (HCP) crystal structure resulting in relatively low ductility (compared to face centered cubic (FCC) and body-centered cubic (BCC) alloys). Several commercial magnesium-based alloys have been developed that include Mg-Al-Zn (AZ-type alloys), Mg-Zn-Mn (ZM-type alloys) and their variants containing additional elements such as rare earth (RE) elements to achieve improved strength, ductility and corrosion resistance. However, the corrosion resistance of the aforementioned alloys is limited due to presence of cathodic second phase particles (or precipitates) that promote galvanic coupling resulting in dissolution of the matrix. Second-phase particles can provide resistance to grain growth during annealing and other heat treatments.

Patent Application WO 2011/051424 A1 relates to biodegradable implantable medical device, in particular an endoprosthesis body formed at least partly from a constructional material comprising deformable super-pure magnesium or alloy thereof further comprising one or more super- pure alloying elements.

Patent Application US 2009/0306765 A1 describes an endoprosthesis that cab include a body including an underlying portion and a surface portion overlying the underlying portion. The underlying portion can include a bioerodible metal in the form of a matrix and corrosion enhancing deposits within the matrix.

Patent Application EP 2169090 A1 describes an implant made in total or in parts of a biodegradable magnesium alloy consisting of Y: 2.0 - 6.0% by weight, Nd: 1.5 - 4.5% by weight, Gd: 0 - 4.0% by weight, Dy: 0 - 4.0% by weight, Er: 0 - 4.0% by weight, Zr: 0.1 - 1.0% by weight, Li:0 - 0.2% by weight, Al: 0 - 0.3% by weight, under the condition that a) a total content of Er, Gd and Dy is in the range of 0.5 - 4.0% by weight and b) a total content of Nd, Er, Gd and Dy is in the range of 2.0 - 5.5% by weight, the balance being magnesium and incidental impurities up to a total of 0.3% by weight.

### SUMMARY

It is desirable to achieve uniform single phase microstructure (without any second phases) to prevent internal galvanic coupling and thereby achieve superior corrosion resistance. In addition to galvanic coupling, impurities present in the Mg-based alloys may play a crucial role in driving corrosion. Elements such as Fe, Ni and Cu should be minimized in the system to improve corrosion. Hence, it is desirable to identify alloying elements that getters the aforementioned impurities. In the absence of any second phase particles providing precipitation strengthening, the single phase alloy will have to rely on other strengthening sources such as solid solution strengthening, grain refinement, and cold work (dislocation strengthening). It is noted that grain refinement improves corrosion resistance and ductility.

In a multiphase system, it is desirable to ensure that the discrete 2^{nd} phase is relatively more electronegative compared to the matrix phase to slow corrosion of the matrix. Alloying elements that could (i) balance the difference in electrochemical potential between matrix and discrete 2^{nd} phase particles and/or (ii) increase the electro positivity of the interconnected matrix relative to the discrete 2^{nd} phase can be added. In addition to galvanic coupling, impurities present in the Mg-based alloys may play a crucial role in driving corrosion. Elements such as Fe, Ni and Cu should be minimized in the system to improve corrosion resistance.

In accordance with the present invention, there is provided a stent comprising a magnesium alloy as defined by the claims.

In an embodiment, a stent is formed from a magnesium alloy, the magnesium alloy consisting essentially of: about 1 weight % Sc; about 0.5 weight % Y; about 1 weight % Li; and balance Mg.

In an embodiment, which is not according to the invention, a stent is formed from a magnesium alloy, the magnesium alloy consisting essentially of: about 1 weight % Sc; about 0.8 weight % Y; and balance Mg.

In an example, which is not according to the invention, a stent is formed from a magnesium alloy, the magnesium alloy consisting essentially of: about 1.5 weight % Sc; about 0.7 weight % Li; and balance Mg.

In an example, which is not according to the invention, a stent is formed from a magnesium alloy, the magnesium alloy consisting essentially of: about 1.5 weight % Y; about 0.7 weight % Li; and balance Mg.

In accordance with an aspect of embodiments, not according to the invention, there is provided a stent comprising a magnesium alloy. The magnesium alloy consists essentially of: 0-5 weight % rare earth element; 0-8 weight % Li; 0-1 weight % Mn; 0-1 weight % Sn; 0-3 weight % Al; 0-4 weight % Zn; and Mg for the balance. The rare earth element may be selected from the group consisting of: Sc, Y, La, Gd, and Nd.

In an example, which is not according to the invention, a stent is formed from a magnesium alloy, the magnesium alloy consisting essentially of: about 3 weight % Li; about 1 weight % Al; and balance Mg.

In the following the words "invention" and "embodiments" are used and/or features are presented as optional. This should be interpreted in such way that protection is sought for the invention as claimed.

### BRIEF DESCRIPTION OF THE DRAWINGS

Figure 1 illustrates a stent in accordance with embodiments of the present invention;
Figures 2A and 2B are phase diagrams based on Scheil calculations and equilibrium calculations, respectively, for an alloy in accordance with an embodiment of the present invention;
Figures 3A and 3B are phase diagrams based on Scheil calculations and equilibrium calculations, respectively, for an alloy in accordance with an embodiment of the present invention;
Figure 4 is a phase diagram based on Scheil calculations for the alloy represented in Figures 2A and 2B, with a modified melting step;
Figures 5A and 5B are phase diagrams based on Scheil calculations and equilibrium calculations, respectively, for an alloy in accordance with an embodiment of the present invention;
Figures 6A and 6B are phase diagrams based on Scheil calculations and equilibrium calculations, respectively, for an alloy in accordance with an embodiment of the present invention;
Figures 7A and 7B are phase diagrams based on Scheil calculations and equilibrium calculations, respectively, for an alloy in accordance with an embodiment of the present invention;
Figures 8A and 8B are phase diagrams based on Scheil calculations and equilibrium calculations, respectively, for an alloy in accordance with an embodiment of the present invention;
Figures 9A and 9B are phase diagrams based on Scheil calculations and equilibrium calculations, respectively, for an alloy in accordance with an embodiment of the present invention;
Figures 10A and 10B are phase diagrams based on Scheil calculations and equilibrium calculations, respectively, for an alloy in accordance with an embodiment of the present invention; and
Figures 11A and 11B are phase diagrams based on Scheil calculations and equilibrium calculations, respectively, for an alloy in accordance with an embodiment of the present invention
Figures 12A and 12B are phase diagrams based on Scheil calculations and equilibrium calculations, respectively, for an alloy in accordance with an embodiment of the present invention;
Figures 13A and 13B are phase diagrams based on Scheil calculations and equilibrium calculations, respectively, for an alloy in accordance with an embodiment of the present invention;
Figures 14A and 14B are phase diagrams for a body centered cubic (BCC) phase and a hexagonally close packed phase (HCP), respectively, of the alloy represented in Figures 13A and 13B;
Figures 15A and 15B are phase diagrams based on Scheil calculations and equilibrium calculations, respectively, for an alloy in accordance with an embodiment of the present invention;
Figures 16A and 16B are phase diagrams for a BCC phase and an HCP phase, respectively, of the alloy represented in Figures 15A and 15B;
Figures 17A and 17B are phase diagrams based on Scheil calculations and equilibrium calculations, respectively, for an alloy in accordance with an embodiment of the present invention;
Figures 18A and 18B are phase diagrams for an Al-Li phase and an HCP phase, respectively, of the alloy represented in Figures 17A and 17B;
Figures 19A and 19B are phase diagrams based on Scheil calculations and equilibrium calculations, respectively, for an alloy in accordance with an embodiment of the present invention;
Figures 20A and 20B a are phase diagrams based on Scheil calculations and equilibrium calculations, respectively, for an alloy in accordance with an embodiment of the present invention; and
Figures 21A and 21B are phase diagrams for an Al-Li phase and an HCP phase, respectively, of the alloy represented in Figures 20A and 20B.

### DETAILED DESCRIPTION

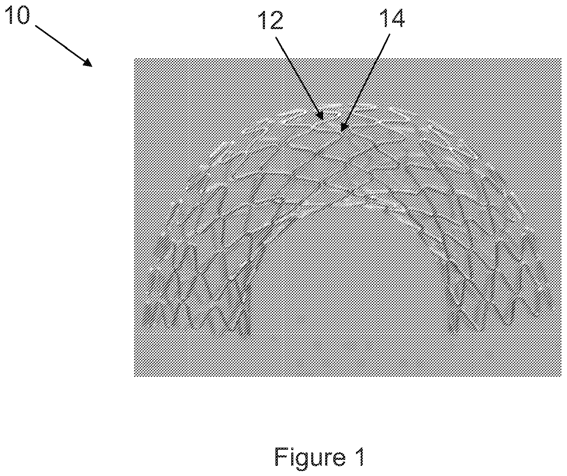

Figure 1 illustrates a stent 10 that includes a plurality of struts 12 and a plurality of crowns or turns 14, with each crown or turn 14 connecting a pair of adjacent struts 12. The stent 10 may be formed from a tube by methods known in the art, such as laser cutting. The tube used to form the stent 10 may be made in accordance with embodiments of the present invention disclosed herein. In an embodiment a single wire may be used to form the plurality of struts 12 and the plurality of crowns or turns 14 by know methods, and the wire used to form the stent 10 may be made in accordance with embodiments of the present invention.

Embodiments of the present invention are directed to a novel Mg-based composition combined with a special recipe for processing to achieve single microstructure with superior corrosion resistance. Additionally, preliminary evaluations reveal good strength and ductility when compared against pure Mg.

The RE elements include Sc, Y, La, Gd, Nd. Mg-Li-RE with Mn and/or Zr is a novel composition wherein the alloying elements are specifically chosen to achieve single phase corrosion-resistant microstructure with minimal impurities. The Mg-Li-RE-Mn-Zr based alloy with Li demonstrates improved ductility as Li is known to activate several additional slip systems in HCP-Mg during deformation. Additionally, Li contributes to solid solution strengthening. RE elements such as Sc and Y promote grain refinement, and due to their high solubility in HCP-Mg, will also improve solid solution strengthening. It was also observed that Sc could getter impurities such as Fe during melting to form second phase (Fe₂Sc) that could sediment to the bottom of the melt. Subsequently, a small portion at the bottom of melt-pool in the crucible may be avoided when pouring to the mold to ensure that gettered Fe-rich phase is removed. This is one of the unique melting strategies that may be followed to improve corrosion resistance.

Mn and Zr in the system may assist in gettering impurities. Additionally, Mn and Zr may promote grain refinement. Initial evaluations using thermodynamic and property models indicate the alloy composition to demonstrate enhanced corrosion resistance, strength and ductility compared to incumbent Mg-based alloys. Possible alloy compositions (all compositions in weight %) are listed in Table I:

**Table I: Single Phase Mg Alloys (examples 6-8 and 12-19 are not according to the invention)**

| | Sc | Y | Li | Mn | Zr | Mg | Hold period for melt (Temperature and Time) |
|---|---|---|---|---|---|---|---|
| Example 1 | 3.5 | 2.6 | - | - | - | Bal | - |
| Example 2 | 4.5 | 2.2 | 1 | - | - | Bal | - |
| Example 3 | 3.5 | 2.6 | - | - | - | Bal | 700°C, <1 hour |
| Example 4 | 4.5 | 2.2 | 1 | - | - | Bal | 700°C, <1 hour |
| Example 5 | 3.5 | 2.6 | - | - | 0.3 | Bal | - |
| Example 6 | - | 3.5 | - | - | - | Bal | - |
| Example 7 | - | 3.5 | - | - | 0.3 | Bal | - |
| Example 8 | - | 3.5 | 1 | - | | Bal | - |
| Example 9 | 1 | 0.8 | | - | - | Bal | |
| Example 10 | 1 | 0.5 | 1 | - | - | Bal | |
| Example 11 | 1 | 0.8 | - | - | - | Bal | 700°C, < 1 hour |
| Example 12 | 1.5 | - | 0.7 | - | - | Bal | |
| Example 13 | - | 2 | - | - | - | Bal | |
| Example 14 | - | 2 | - | - | 0.3 | Bal | |
| Example 15 | - | 1.5 | 0.7 | - | - | Bal | |
| Example 16 | - | 2 | 3 | 0.4 | - | Bal | |
| Example 17 | 3 | - | 0.5 | - | 0.3 | Bal | |
| Example 18 | 2 | - | 1 | - | 0.3 | Bal | |
| Example 19 | 4 | - | 0.2 | - | 0.3 | Bal | |

Hence, it is desirable to identify alloying elements that getters the aforementioned impurities. In the multiphase system, precipitates (or discrete particles) can contribute to strengthening by acting as shearable or Orowan obstacles. Additional sources of strengthening include solid solution strengthening, grain refinement, and cold work (dislocation strengthening). It is noted that grain refinement improves corrosion resistance and ductility.

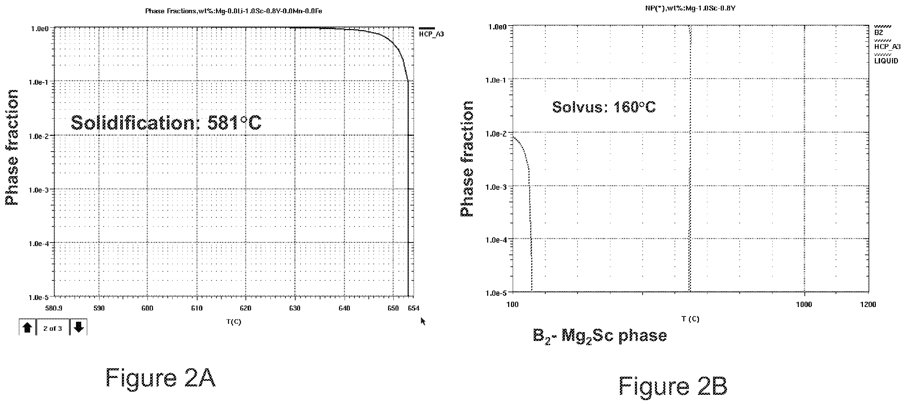

Figures 2A and 2B are Scheil and equilibrium diagrams, respectively, for an alloy in accordance with Example 9, which is an alloy having 1 wt.% Sc, 0.8 wt.% Y, and Mg as the balance. The as-cast microstructure is expected to be single phase. As illustrated in Figure 2A, the solidification temperature using Scheil calculations is about 581°C, and the solvus using equilibrium calculations is about 160°C, as illustrated in Figure 2B. The low solvus temperature should ensure sufficient room for solution heat treatment (SHT), if needed. No precipitates are expected to form during the processing stage. After melting the alloy in a non-steel crucible, such as a Mo-crucible, homogenization to dissolve all eutectic products may be completed at 525°C for 10 hours, then the melt may be extruded at 300°C and the extrusion ratio may be greater than 45 to obtain fine grin sizes, then a tube may be drawn at 300°C to ensure no second phases, and if needed, SHT/annealing may be completed at 350° for 2-4 hours.

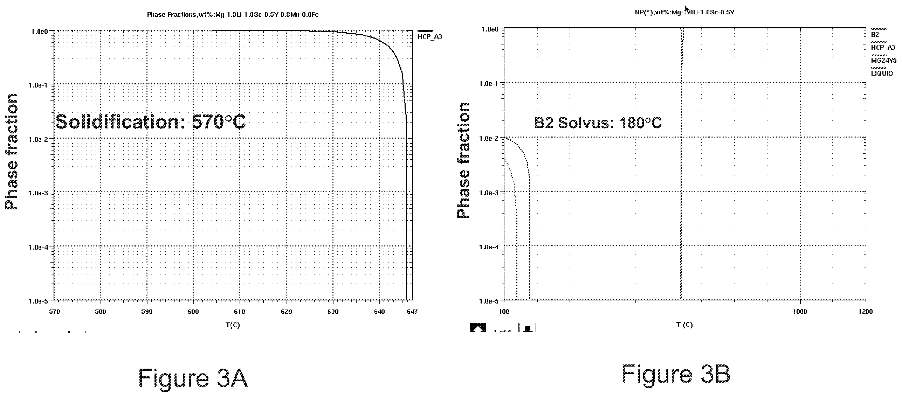

Figures 3A and 3B are Scheil and equilibrium diagrams, respectively, for an alloy in accordance with Example 10, which is an alloy having 1 wt % Sc, 0.5 wt% Y, 1 wt.% Li, and Mg as the balance. The as-cast microstructure is expected to be single phase. As illustrated in Figure 3A, the solidification temperature using Scheil calculations is about 570°C, and the B2 solvus using equilibrium calculations is about 180°C, as illustrated in Figure 3B. B2 and Mg₂₄Y₅ are potential equilibrium phases (low solvus). After melting, homogenization may be completed at 525°C for 10 hours, then the melt may be extruded at 300°C and the extrusion ratio may be greater than 45, then a tube may be drawn at 300°C to ensure no second phases, and if needed, SHT/annealing may be completed at 350° for 2-4 hours.

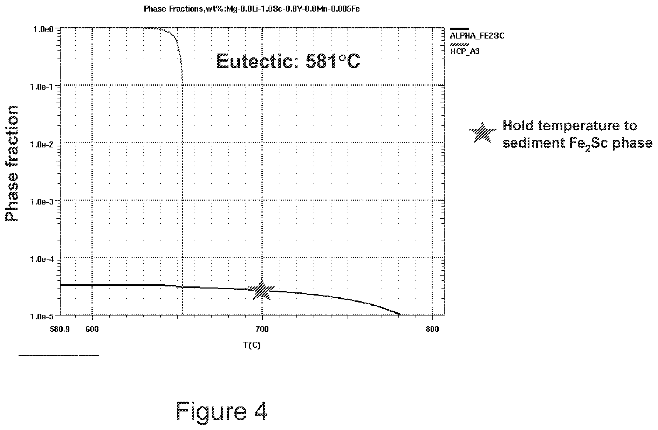

Figure 4 is a Scheil diagram for the alloy represented in accordance with Example 9, with a modified melting step, which is represented as Example 11 in Table I. As illustrated in Figure 4, the eutectic temperature using Scheil calculations is about 581°C. The solvus using equilibrium calculations is expected to be about 160°C In this Example, two-step melting is expected. In step 1, the alloy will be melted and held at 700°C for 30 minutes, then poured into another crucible so that the bottom portion, which may be up to 10% of the melt, may be discarded due to sediment In step 2, the remaining material may be re-melted and poured into a mold at 800°C. Homogenization may be completed at 525°C for 10 hours, then the melt may be extruded at 300°C and the extrusion ratio may be greater than 45, then a tube may be drawn at 300°C to ensure no second phases, and if needed, SHT/annealing may be completed at 350° for 2-4 hours.

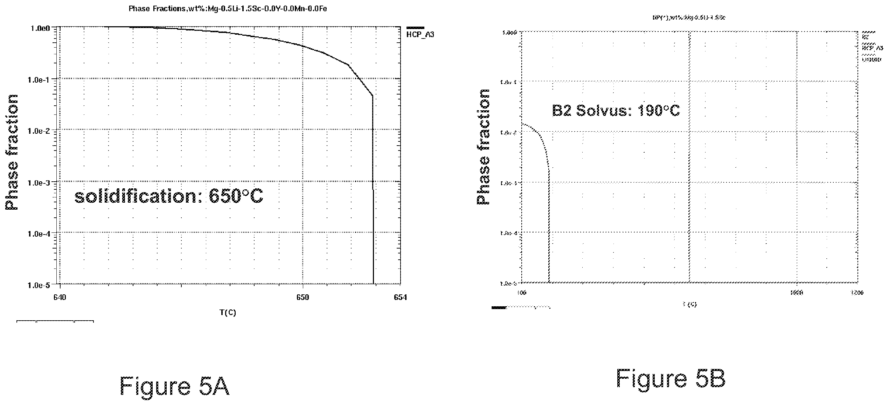

Figures 5A and 5B are Scheil and equilibrium diagrams, respectively, for an alloy in accordance with Example 12, which is an alloy having 1.5 wt % Sc, 0.7 wt.% Li, and Mg as the balance. The as-cast microstructure is expected to be single phase. As illustrated in Figure 5A, the solidification temperature using Scheil calculations is about 650°C, and the B2 solvus using equilibrium calculations is about 190°C, as illustrated in Figure 5B. After melting, homogenization may be completed at 525°C for 10 hours, then the melt may be extruded at 300°C and the extrusion ratio may be greater than 45, then a tube may be drawn at 300°C to ensure no second phases, and if needed, SHT/annealing may be completed at 350° for 2-4 hours.

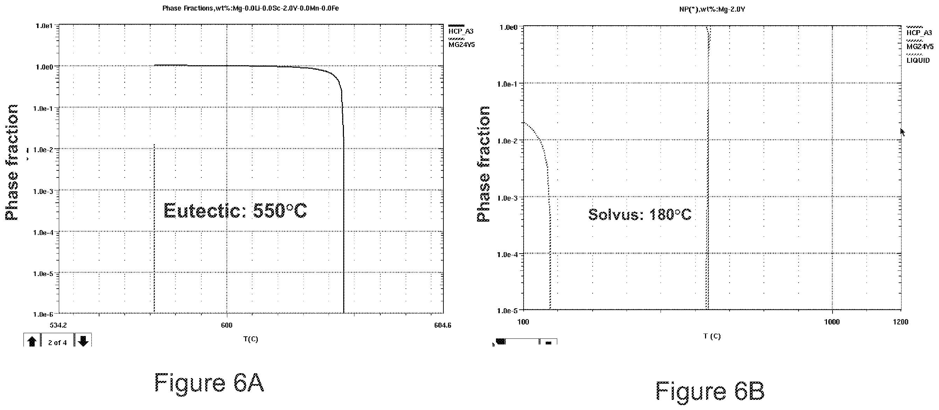

Figures 6A and 6B are Scheil and equilibrium diagrams, respectively, for an alloy in accordance with Example 13, which is an alloy having 2 wt.% Y, and Mg as the balance. The as-cast microstructure is expected to have some Mg₂₄Y₅. As illustrated in Figure 6A, the eutectic temperature using Scheil calculations is about 550°C, and the solvus using equilibrium calculations is about 180°C, as illustrated in Figure 6B. After melting, homogenization may be completed at 500°C for 10 hours, then the melt may be extruded at 300°C and the extrusion ratio may be greater than 45, then a tube may be drawn at 300°C to ensure no second phases, and if needed, SHT/annealing may be completed at 350° for 4 hours.

Example 14, which is an alloy having 2 wt.% Y, 0.3 wt.% Zr, and Mg as the balance, is designed to explore the effect of Zr, which could be an efficient grain refiner (innoculant) and has been reported to enhance corrosion resistance. Zr is also reported to getter impurities such as Fe in Mg-based alloys. The expected eutectic temperature is greater than 550°C, and the expected solvus temperature is expected to be 180°C (based on Mg₂₄Y₅). After melting, homogenization may be completed at 500°C for 10 hours, then the melt may be extruded at 300°C and the extrusion ratio may be greater than 45, then a tube may be drawn at 300°C (Zr innoculant may be present as second phase in the system), and if needed, SHT/annealing may be completed at 350° for 4 hours.

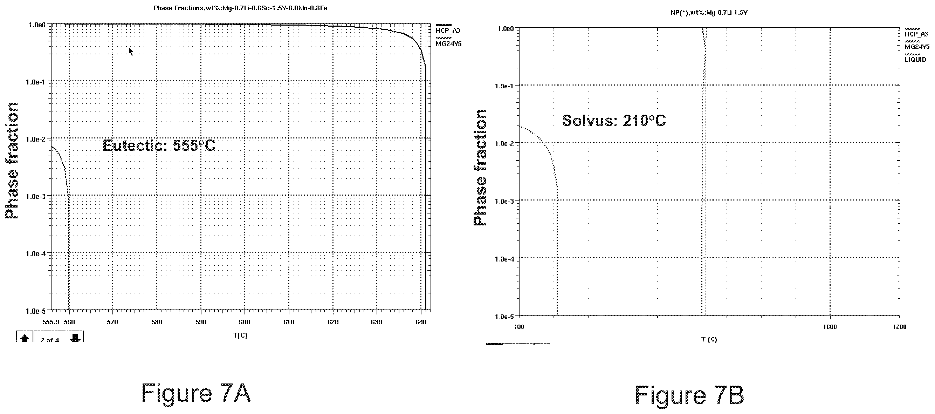

Figures 7A and 7B are Scheil and equilibrium diagrams, respectively, for an alloy in accordance with Example 15, which is an alloy having 1.5 wt % Y, 0.7 wt.% Li, and Mg as the balance. The as-cast microstructure is not expected to be single phase, but there should be sufficient room for homogenization. As illustrated in Figure 7A, the eutectic temperature using Scheil calculations is about 555°C, and the solvus using equilibrium calculations is about 210°C, as illustrated in Figure 7B. After melting, homogenization may be completed at 500°C for 10 hours, then the melt may be extruded at 300°C and the extrusion ratio may be greater than 45, then a tube may be drawn at 300°C (no second phases expected), and if needed, SHT/annealing may be completed at 350° for 2-4 hours.

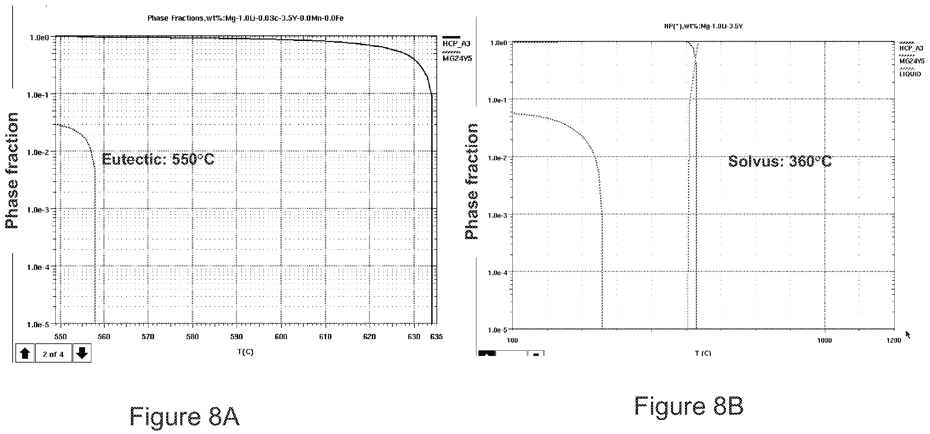

Figures 8A and 8B are Scheil and equilibrium diagrams, respectively, for an alloy in accordance with Example 8, which is an alloy having 3.5 wt.% Y, 1 wt.% Li, and Mg as the balance. As illustrated in Figure 8A, the eutectic temperature using Scheil calculations is about 550°C, and the solvus using equilibrium calculations is about 360°C, as illustrated in Figure 8B. The high solvus temperature is expected to assist with grain refinement during extrusion and tube drawings. After melting, homogenization may be completed at 500°C for 10 hours, then the melt may be extruded at 325°C and the extrusion ratio may be greater than 45, then a tube may be drawn at 300°C (second phase expected, assisting grain pinning), and if needed, SHT/annealing may be completed at 400° for 4 hours.

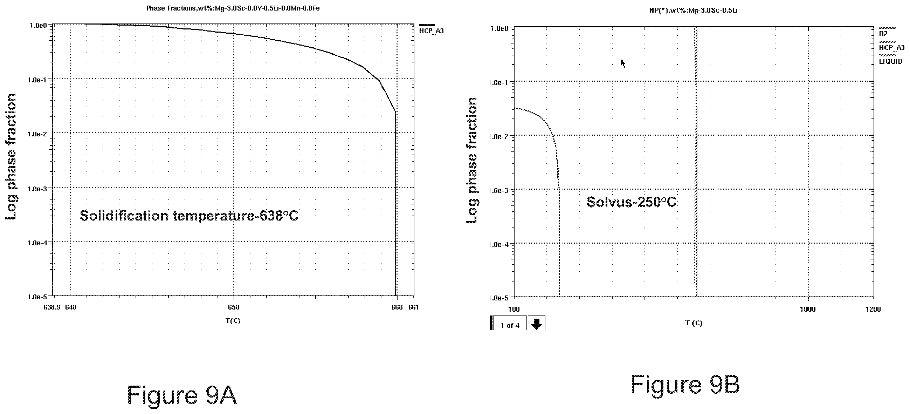

Figures 9A and 9B are Scheil and equilibrium diagrams, respectively, for an alloy in accordance with Example 17, which is an alloy having 3 wt.% Sc, 0.5 wt.% Li, 0.3 wt.% Zr, and Mg as the balance. Because Zr was not in the thermodynamic database used for the Scheil and equilibrium calculations, Figures 9A and 9B were actually generated for Mg-Sc-Li. As illustrated in Figure 9A, the solidification temperature using Scheil calculations is about 638°C, and the solvus using equilibrium calculations is about 250°C (based on B2 phase), as illustrated in Figure 9B. The eutectic temperature of the alloy with Zr is expected to be greater than 550°C, and the solvus temperature of the alloy with Zr is expected to be 250°C. The as-cast microstructure may not be truly single phase due to the presence of Zr to act as innoculant. Adequate room for homogenization is expected. After melting, homogenization may be completed at 500°C for 10 hours, then the melt may be extruded at 300°C and the extrusion ratio may be greater than 45, then a tube may be drawn at 300°C (Zr innoculant present as second phase in the system), and if needed, SHT/annealing may be completed at 350° for 4 hours.

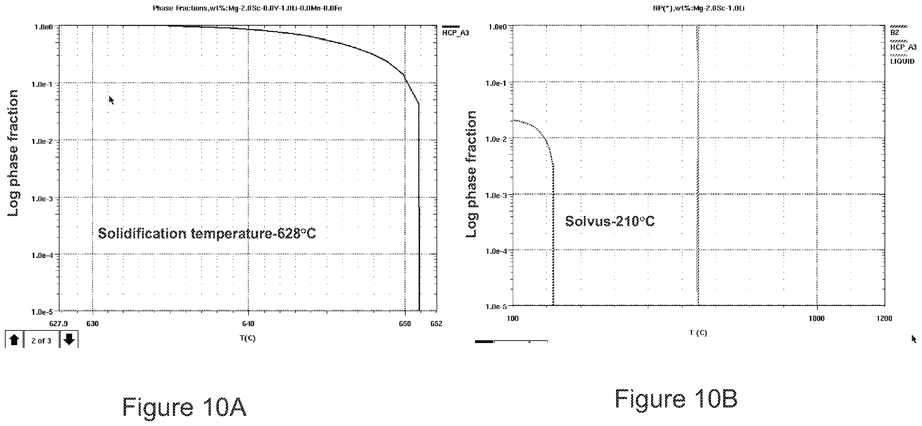

Figures 10A and 10B are Scheil and equilibrium diagrams, respectively, for an alloy in accordance with Example 18, which is an alloy having 2 wt.% Sc, 1 wt.% Li, 0.3 wt.% Zr, and Mg as the balance. Because Zr was not in the thermodynamic database used for the Scheil and equilibrium calculations, Figures 10A and 10B were actually generated for Mg-Sc-Li. As illustrated in Figure 10A, the solidification temperature using Scheil calculations is about 628°C, and the solvus using equilibrium calculations is about 210°C (based on B2 phase), as illustrated in Figure 10B. The eutectic temperature of the alloy with Zr is expected to be greater than 550°C, and the solvus temperature of the alloy with Zr is expected to be 210°C. The as-cast microstructure may not be truly single phase due to the presence of Zr to act as innoculant. Adequate room for homogenization is expected. After melting, homogenization may be completed at 500°C for 10 hours, then the melt may be extruded at 300°C and the extrusion ratio may be greater than 45, then a tube may be drawn at 300°C (Zr innoculant present as second phase in the system), and if needed, SHT/annealing may be completed at 350° for 4 hours.

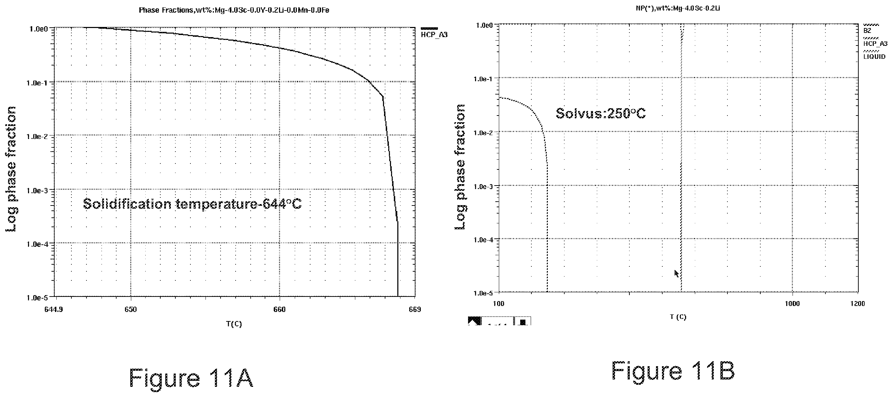

Figures 11A and 11B are Scheil and equilibrium diagrams, respectively, for an alloy in accordance with Example 19, which is an alloy having 4 wt.% Sc, 0.2 wt.% Li, 0.3 wt.% Zr, and Mg as the balance. Because Zr was not in the thermodynamic database used for the Scheil and equilibrium calculations, Figures 11A and 11B were actually generated for Mg-Sc-Li. As illustrated in Figure 11A, the solidification temperature using Scheil calculations is about 644°C, and the solvus using equilibrium calculations is about 250°C (based on B2 phase), as illustrated in Figure 11B. The eutectic temperature of the alloy with Zr is expected to be greater than 550°C, and the solvus temperature of the alloy with Zr is expected to be 250°C. The as-cast microstructure may not be truly single phase due to the presence of Zr to act as innoculant. Adequate room for homogenization is expected. After melting, homogenization may be completed at 500°C for 10 hours, then the melt may be extruded at 300°C and the extrusion ratio may be greater than 45, then a tube may be drawn at 300°C (Zr innoculant present as second phase in the system), and if needed, SHT/annealing may be completed at 350° for 4 hours.

### Two-Phase Alloys

Embodiments of the present invention are also directed to novel Mg-based compositions with a novel recipe for processing to achieve two phase microstructure with a more anodic discrete phase (distributed) relative to a more cathodic matrix (or interconnected) phase. The anodic discrete phase in this case may impart corrosion resistance to the continuous interconnected Mg-rich matrix by behaving as a sacrificial anode, and may dissolve (corrode) preferentially to the matrix phase. The Alloying elements are defined by the claims.

The RE elements include Sc, Y, La, Gd, Nd. Mg-Li-RE-Al-Zn-Mn is a novel composition space wherein the alloying elements and respective weight percentage are specifically chosen to achieve two phase corrosion-resistant microstructure with the discrete phase being more anodic. The compositions should ensure formation of interconnected HCP Mg-Li phase with varying second phase particles. The addition of Li may contribute to solid solution strengthening and enhanced ductility. RE elements such as Sc and Y may also improve solid solution strengthening.

The addition of Nd to HCP Mg-Li may promote formation of a BCC Mg-Li phase, which is expected to be relatively electronegative (i.e. anodic) compared to an HCP matrix. Certain compositions containing Zn may require a novel process path in which the alloy will be homogenized at low temperature and after thermomechanical processing such as extrusion, should be tempered/aged at higher temperature (relative to homogenization temperature) to promote formation of anodic second phases. Al-containing alloys promote precipitation of Al-Li phase in HCP Mg-Li matrix. Al-Li phase is expected to be anodic relative to the matrix thereby reducing the corrosion rate of the alloy. The phase fraction of the anodic second phase could be up to 30%. Additionally, A1 may contribute to solid solution strengthening. Sn is known to be highly cathodic and presence of Sn in the HCP Mg matrix solid solution is expected to make the matrix cathodic relative to the precipitate phase. Certain compositions are designed to ensure the precipitate phase is present during thermomechanical processing at intermediate temperature to assist in grain pinning. Mn is expected to assist in gettering impurities during melting. Potential compositions (all compositions in weight %) are listed in Table II below:

**Table II: Two Phase Mg Alloys (all not according to the invention)**

| | Li | Mn | Sn | Al | Zn | Nd | Mg |
|---|---|---|---|---|---|---|---|
| Example 20 | 5 | - | - | - | 2 | - | Bal |
| Example 21 | 5 | 0.4 | - | - | 2 | | Bal |
| Example 22 | 4 | - | - | - | - | 0.5 | Bal |
| Example 23 | 5 | - | - | 2 | - | - | Bal |
| Example 24 | 3 | - | - | 1 | - | - | Bal |
| Example 25 | 4 | - | 0.4 | 1.5 | - | - | Bal |
| Example 26 | 6 | 0.5 | - | - | 1.5 | - | Bal |
| Example 27 | 6.2 | - | 1 | - | - | - | Bal |
| Example 28 | 4 | - | - | - | - | 1 | Bal |
| Example 29 | 4 | 0.2 | - | - | - | 1 | Bal |
| Example 30 | 5 | - | 0.3 | - | 1.5 | 0.5 | Bal |

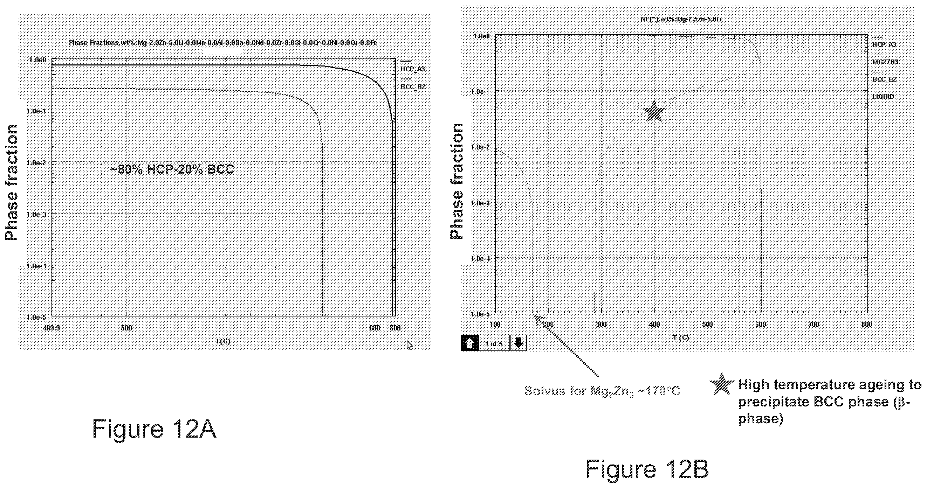

Figures 12A and 12B are Scheil and equilibrium diagrams, respectively, for an alloy in accordance with Example 20, which is an alloy having 5 wt.% Li, 2 wt.% Zn, and Mg as the balance. As illustrated in Figure 12A, the eutectic temperature using Scheil calculations is about 470°C, and the solvus for Mg₂Zn₃ using equilibrium calculations is about 170°C. After melting, homogenization may be completed at 250°C for 15 hours to ensure a single phase microstructure during processing (extrusion, tube drawing, etc.). The melt may then be extruded at 325°C and the extrusion ratio may be greater than 45, then a tube may be drawn at 300°C during which BCC second phase is expected, and if needed, SHT/annealing may be completed at 400° for 6 hours. The α-phase (HCP) will form prior to formation of the β-phase (BCC). High temperature ageing of tube drawn specimen should promote precipitation of BCC phase in HCP matrix, which is the desired microstructure. Unlike the common practice, the alloy of this Example needs to be solutionized at a relatively low temperature (∼250°C) and tempered at a higher temperature (∼400°C), which is a novel heat treatment.

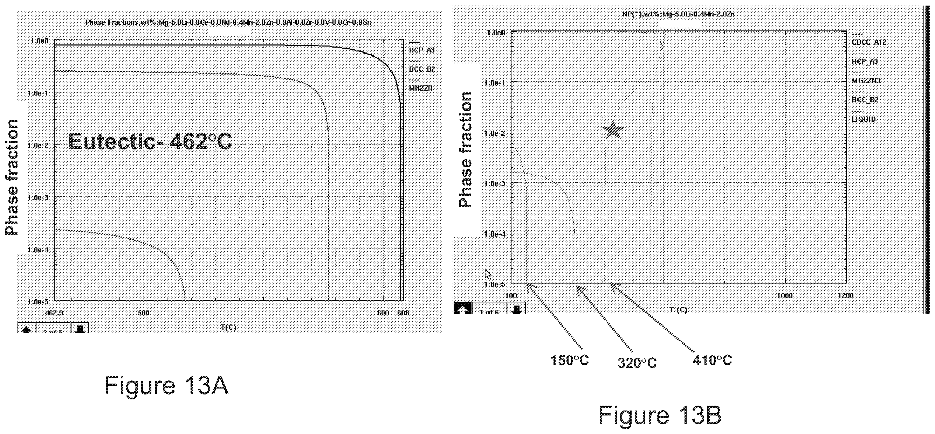

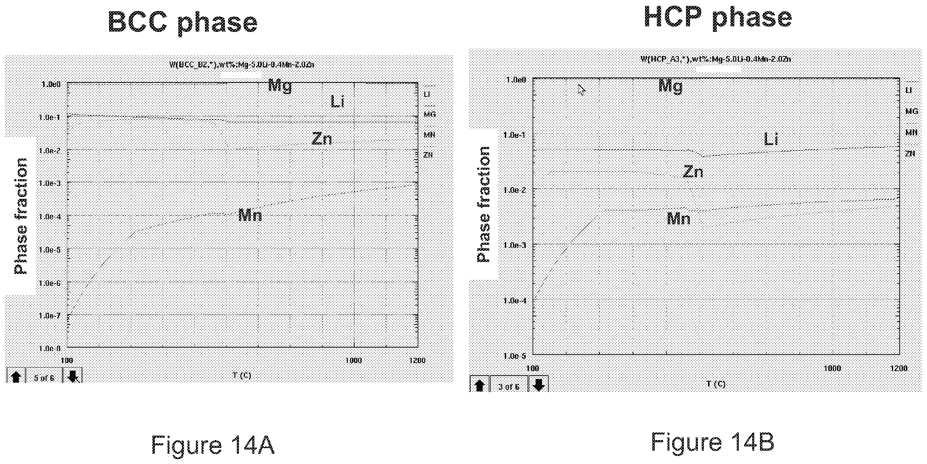

Figures 13A and 13B are Scheil and equilibrium diagrams, respectively, and Figures 14A and 14B are phase diagrams for the BCC phase and the HCP, respectively, of the alloy of Example 21, which is an alloy having 5 wt.% Li, 0.4 wt.% Mn, 2 wt.% Zn, and Mg as the balance. As illustrated in Figure 13A, the eutectic temperature using Scheil calculations is about 462°C, and the solvus for BCC_A12 (Mg-Mn) using equilibrium calculations is about 320°C. After melting, homogenization may be completed at 410°C for 15 hours. The melt may then be extruded at 300°C and the extrusion ratio may be greater than 45, then a tube may be drawn at 300°C, and SHT/annealing may be completed at 350° for 2-4 hours. After annealing, tempering or ageing may be completed at 410°C for 6 hours. The relatively low eutectic temperature and narrow window (100°C) for homogenization and narrow window for ageing (∼420°C) may enable formation of a 1-2% phase fraction of BCC. The presence of BCC_A12 (Mg-Mn) phase may assist in grain refinement during extrusion, which may be an advantage Also, the ageing treatment may ensure that all Mn is put back into the HCP solid solution, which may assist in balancing the difference in electrochemical potential.

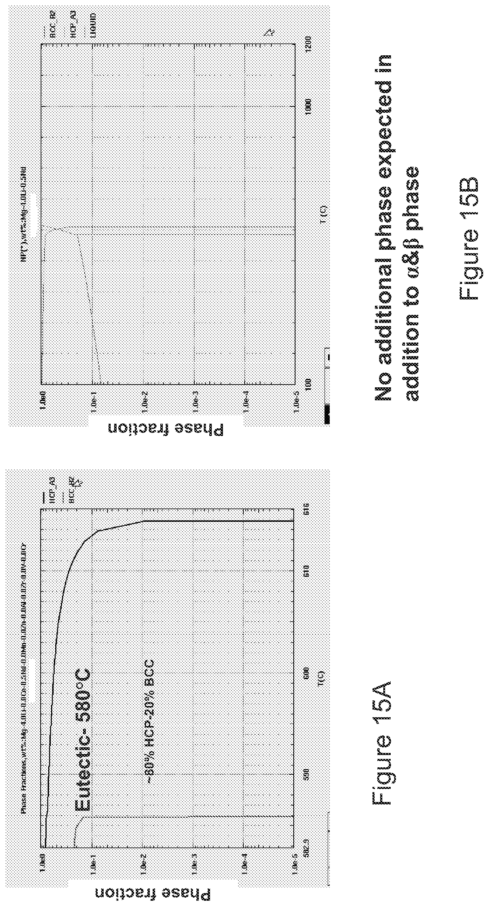

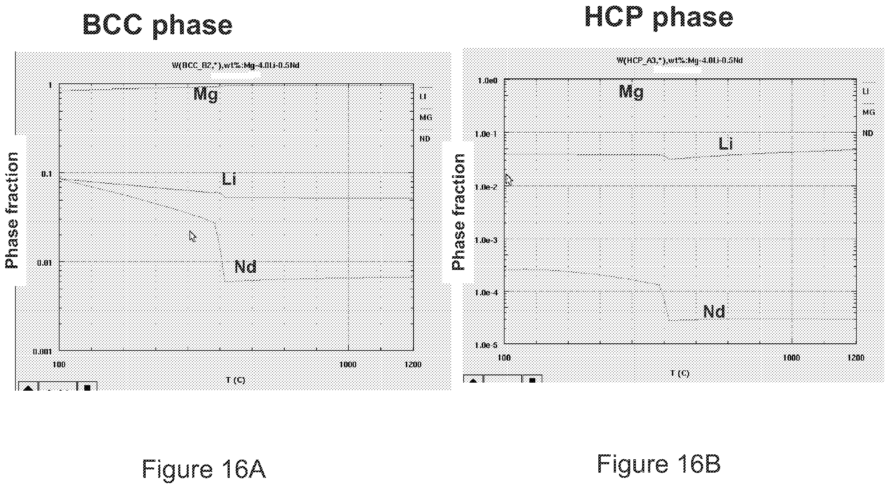

Figures 15A and 15B are Scheil and equilibrium diagrams, respectively, and Figures 16A and 16B are phase diagrams for the BCC phase and the HCP phase, respectively, of the alloy of Example 22, which is an alloy having 4 wt.% Li, 0.5 wt.% Nd, and Mg as the balance. As illustrated in Figure 15A, the eutectic temperature using Scheil calculations is about 580°C, with 80% HCP and 20% BCC, and no additional phase is expected in addition to the α-phase and β-phase, as illustrated in Figure 15B. Figure 16A illustrates higher Li in the BCC phase, and Nd is present in the BCC phase. Based on the thermodynamic calculations, the phase fraction of β is expected to be in the range of 7% - 20% (assuming low enough to cause minimal structural instability after corrosion). The β-phase is expected to act as an anode by virtue of the higher Li content. After melting, homogenization may be completed at 500°C for 10 hours, then the melt may be extruded at 300°C and the extrusion ratio may be greater than 45, then a tube may be drawn at 300°C to ensure no second phases, and if needed, SHT/annealing may be completed at 300° for 6 hours.

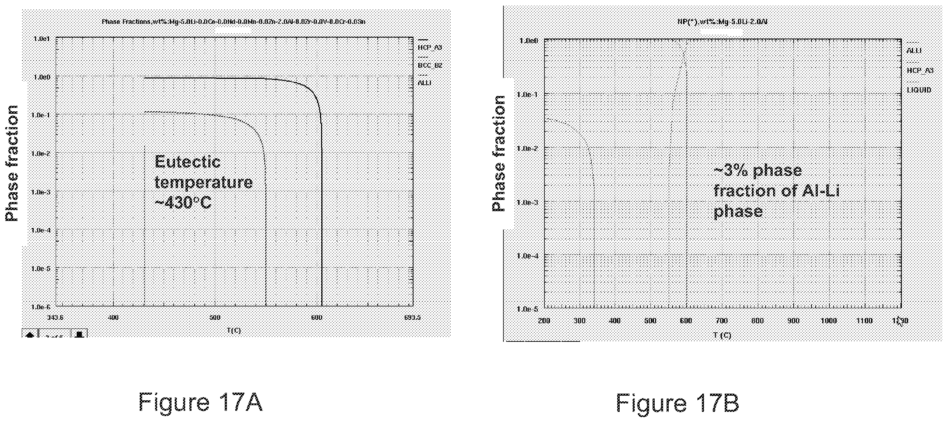

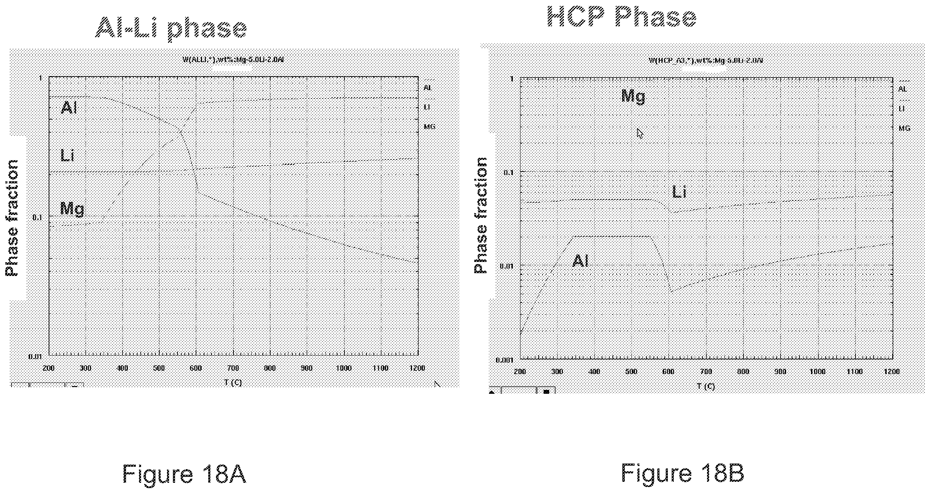

Figures 17A and 17B are Scheil and equilibrium diagrams, respectively, and Figures 18A and 18B are phase diagrams for an Al-Li phase and an HCP phase, respectively, for an alloy in accordance with Example 23, which is an alloy having 5 wt% Li, 2 wt.% Al, and Mg as the balance. As illustrated in Figure 17A, the eutectic temperature is about 430°C, which is somewhat low and may mean a small window for homogenization (∼380°C), which may translate to longer homogenization time. As illustrated in Figure 17B, the solvus temperature (Al-Li) is about 350°C, and there is about a 3% phase fraction of the Al-Li phase. The Al-Li second phase is expected to be anodic and drive corrosion, and has the potential to demonstrate higher electro negativity compared to HCP matrix by virtue of high Li content. After melting, homogenization may be completed at 390°C for 15 hours, then the melt may be extruded at 300°C and the extrusion ratio may be greater than 45, then a tube may be drawn at 300°C during which time the Al-Li phase is expected to act as a grain pinning agent, annealing may be completed at 350°, and tempering may be completed at 300°C for 6 hours.

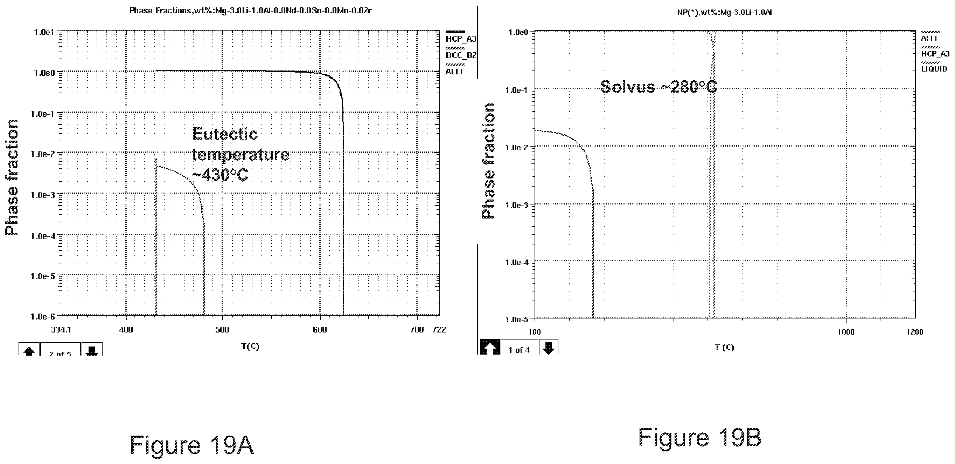

Figures 19A and 19B are Scheil and equilibrium diagrams, respectively, for an alloy in accordance with Example 24, which is an alloy having 3 wt.% Li, 1 wt.% Al, and Mg as the balance. As illustrated in Figure 19A, the eutectic temperature is about 430°C. As illustrated in Figure 19B, the solvus temperature (Al-Li) is about 280°C. The relatively lower phase fraction of Al-Li in the alloy may discern the advantages/limitations of the Al-Li phase. After melting, homogenization may be completed at 380°C for 15 hours, then the melt may be extruded at 300°C and the extrusion ratio may be greater than 45, then a tube may be drawn at 300°C. During extrusion and tube drawing, the Al-Li phase is not expected to contribute to grain refinement Annealing (during tube drawing) may be completed at 350°, and tempering may be completed at 250°C for 8 hours.

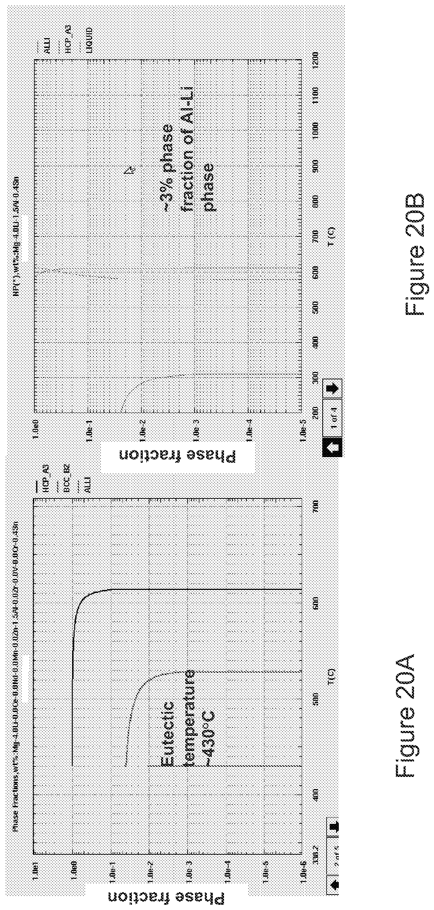

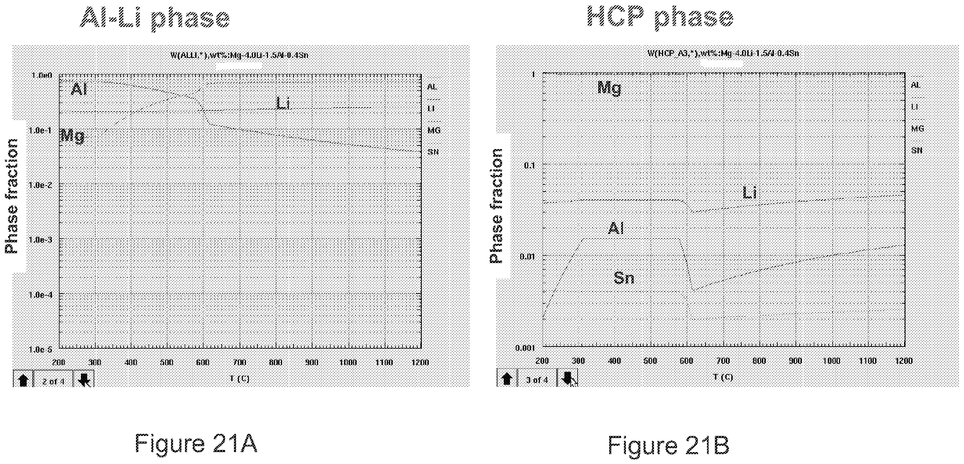

Figures 20A and 20B are Scheil and equilibrium diagrams, respectively, and Figures 21A and 21B are phase diagrams for an Al-Li phase and an HCP phase, respectively, of the alloy in accordance with Example 25, which is an alloy having 4 wt.% Li, 1.5 wt.% Al, 0.4 wt.% Sn, and Mg as the balance. This Example is a variant of the HCP Al-Li concept with the addition of Sn to control the cathodic nature of the matrix. Having 0.4 wt.% Sn should ensure that all Sn can be put into the HCP solid solution. As illustrated in Figure 20A, the eutectic temperature is about 430°C, which is somewhat low and may result in a small homogenization window of about 380°C for several hours. As illustrated in Figure 20B, the solvus temperature (Al-Li) is about 310°C, and there is about a 3% phase fraction of the Al-Li phase. As illustrated in Figure 21B, Sn is present in the HCP solid solution. The extrusion and tube forming temperature is sufficient to dissolve all Sn in HCP (for the selected composition). The presence of Sn may make the matrix cathodic, which may ensure the Al-Li phase corrodes prior to the matrix. After melting, homogenization may be completed at 380°C for 15 hours, then the melt may be extruded at 300°C and the extrusion ratio may be greater than 45, then a tube may be drawn at 300°C. During drawing, the Al-Li phase is expected to act as a grain pinning agent. Annealing (during tube drawing) may be completed at 350°, and tempering may be completed at 280°C for 6 hours.

### Testing of Alloys

Five of the examples above were selected for melting, extrusion, and testing. Specifically, single-phase alloy Examples 9, 10, 12, and 15 and two-phase alloy Example 24 were extruded at 350°C at an extrusion ratio of 20.25 to form extruded rods at least three feet in length. The extrusion dies were lubricated with moly-disulfide and the melts were cooled by being quenched with liquid nitrogen. The actual composition of each sample was measured using inductively coupled plasma mass spectrometry (ICP-MS), and Table III lists the measured compositions (with balance Mg) of the extruded samples, in ppm (except where otherwise noted).

**Table III: Measured Compositions by ICP-MS (in ppm, except where otherwise noted)**

| **Sample** | **Li** | **Sc** | **Y** | **Mo** | **Al** | **Pd** | **Fe** | **Cu** |
|---|---|---|---|---|---|---|---|---|
| Example 9 (1) | 2.7 | 3600 | 3000 | 130 | 16 | <0.5 | 33 | 9.2 |
| Example 9 (2) | 4 | 7000 | 3600 | 33 | 20 | <0.5 | 41 | 5 |
| Example 10 | 1.1 wt% | 8400 | 4400 | 2.5 | 19 | <0.5 | 13 | 1.7 |
| Example 12 | 6300 | 1.4 wt% | 140 | 940 | 16 | 1.5 | 66 | 4.6 |
| Example 15 (1) | 4800 | 1.3 | 1.4 wt% | 1100 | 27 | 1.9 | 99 | 4.4 |
| Example 15 (2) | 6800 | <0.5 | 1.4 wt% | 470 | 25 | <0.5 | 40 | 4.5 |
| Example 24 | 3.7 wt% | 1200 | 2.9 | 27 | 9300 | 0.5 | 16 | 3.8 |

The microstructure of a sample extruded from the Example 10 alloy was also characterized and the composition of the sample was measured by energy-dispersive X-ray spectroscopy. Table IV lists the measured compositions, in weight %, of the matrix and of second phases in the alloy. The second phases present in the alloy are Mg-oxides with limited RE solubility. The oxides are expected to be relatively inert and not significantly affect the corrosion resistance of the alloy.

**Table IV: Example 10 Alloy Measured Compositions by EDX (in weight %)**

| **Point on Sample** | **Mg** | **Sc** | **Y** | **O** |
|---|---|---|---|---|
| 1 | 57.63 | 2.5 | 0.48 | 39.39 |
| 2 | 59.81 | 0.48 | 0.08 | 39.63 |
| 3 | 58.96 | 1.24 | 0.27 | 39.53 |
| 4 (matrix) | 98.71 | 0.87 | 0.42 | - |

One of the extruded rods having the composition of the Example 10 alloy was also further extruded into a wire having a diameter of about 0.15 mm. The extruded wire was tested for mechanical properties, including ultimate tensile strength, yield strength, and elongation, and the results of the mechanical testing, as well as mechanical testing of three commercial Mg alloys are listed in Table V. As shown, the wire made from the Example 10 alloy had improved combined mechanical properties (strength and elongation) as compared to three commercial Mg alloys, in which AE42 is an alloy of Mg with 4 wt% A1 and 2 wt% RE; ZM21 is an alloy of Mg with 2 wt% Zn and 1 wt% Mn, and AZ31 is an alloy of Mg with 3 wt% A1 and 1 wt% Zn.

**Table V: Mechanical Properties of Example 10 and Commercial Mg Alloys**

| **Property** | **AE42** | **ZM21** | **Example 10** | **AZ31** |
|---|---|---|---|---|
| Ultimate Tensile Strength (MPa) | 321 | 328 | 335 | 277 |
| Yield Strength (MPa) | 287 | 305 | 296 | 227 |
| Elongation (%) | 5.1 | 2.7 | 7.4 | 8.9 |
| Diameter (mm) | 0.152 | 0.152 | 0.152 | 0.152 |

Corrosion of a stent that was manufactured from the Example 10 alloy showed improved uniformity, which is desirable for a stent application. The stent made from the Example 10 alloy corroded in a uniform pattern from the outside to the center of the wire, while the commercial alloys listed above had non-uniform corrosion with a high level of pitting. It is estimated that a stent made from the Example 10 alloy will keep its integrity until the end of corrosion and stents made from the commercial alloys listed above will break apart during the corrosion due to pitting and/or localized corrosion.

The microstructure of a sample of the Example 15 alloy was also characterized and the composition of the sample was measured by energy-dispersive X-ray spectroscopy. Table VI lists the measured compositions, in weight %, of the matrix and of second phases in the alloy. The second phases present in the alloy are Mg-oxides and RE-oxides. Two shapes of oxide particles were observed, including circular MgO and elongated MgO + Y₂O₃.

**Table VI: Example 15 Alloy Measured Compositions by EDX (in weight %)**

| **Point on Sample** | **Mg** | **Y** | **O** |
|---|---|---|---|
| 1 | 60.01 | 0.40 | 39.60 |
| 2 | 53.38 | 9.05 | 37.57 |
| 3 | 57.44 | 3.75 | 38.81 |
| 4 (matrix) | 97.92 | 2.08 | - |

The microstructure of a sample of the Example 9 alloy was also characterized. The matrix composition was measured to be close to the nominal composition, and the observed second phases were oxides, including Mg-oxides and RE-oxides. Barring the oxides, the microstructure is single phase (in accordance with predictions).

The microstructure of a sample of the Example 24 (two-phase) alloy was also characterized. The matrix composition was close to the nominal composition, and the observed oxides were Mg-oxides and Al-oxides.

Tables I and II include additional Examples that were not described in detail, but still fall within the scope of the present invention and are claimed below. In addition, Table VII lists additional compositions (all elements in weight %) that may be used to enhance corrosion resistance of stents comprising Mg, and fall within the scope of the present invention. The additional compositions may include 1 to 3.5 wt% Li; 0.25 to 9 wt% Sc; 0.25 to 5 wt% Y 0.5 to 1 wt% Mn; 0.3 to 1 wt% Zr; and balance Mg.

**Table VII: Additional Compositions**

| | Sc | Y | Li | Mn | Zr | Mg |
|---|---|---|---|---|---|---|
| Example 31 | 3 | 0.5 | 2 | - | - | Bal |
| Example 32 | 0.25 | 0.25 | 3 | - | - | Bal |
| Example 33 | 9 | 0.5 | - | 0.5 | - | Bal |
| Example 34 | 0.5 | 5 | - | 0.5 | - | Bal |
| Example 35 | 9 | 0.5 | - | - | 0.6 | Bal |
| Example 36 | 0.5 | 5 | - | - | 0.6 | Bal |
| Example 37 | 0.25 | - | 3 | 1 | - | Bal |
| Example 38 | - | 0.5 | 3 | 0.6 | - | Bal |
| Example 39 | - | 0.5 | 3 | - | 1 | Bal |
| Example 40 | 0.25 | - | 3 | - | 1 | Bal |
| Example 41 | 2 | - | 3.5 | - | - | Bal |

Examples 37 to 41 are not according to the invention.

## Claims

1. A stent formed from a magnesium alloy, the magnesium alloy consisting essentially of:
a rare earth content of up to 10 weight %, the rare earth content consisting of:
0.25-9 weight% Sc; and
0.25-5 weight% Y;
one of:
1-3.5 weight% Li;
0.5-1 weight% Mn;
0.3-1 weight% Zr;
balance Mg.

2. The stent according to claim 1, the magnesium alloy consisting essentially of:
a rare earth content of up to 10 weight %, the rare earth content consisting of:
0.25-9 weight% Sc; and
0.25-5 weight% Y;
one of:
1-3.5 weight% Li;
0.5-1 weight% Mn;
balance Mg.

3. The stent according to claim 1, the magnesium alloy consisting essentially of:
a rare earth content of up to 10 weight %, the rare earth content consisting of:
0.25-9 weight% Sc; and
0.25-5 weight% Y;
1-3.5 weight% Li; and
balance Mg.

4. The stent according to claim 1, wherein the magnesium alloy consists essentially of:
1 weight % Sc;
0.5 weight% Y;
1 weight % Li; and
balance Mg.

## Patentansprüche

1. Stent, der aus einer Magnesiumlegierung gebildet ist, wobei die Magnesiumlegierung im Wesentlichen aus Folgendem besteht:
einem Seltenerdmetallgehalt von bis zu 10 Gew.-%, wobei der Seltenerdmetallgehalt aus Folgendem besteht:
0,25-9 Gew.-% Sc; und
0,25-5 Gew.-% Y;
einem von:
1-3,5 Gew.-% Li;
0,5-1 Gew.-% Mn;
0,3-1 Gew.-% Zr;
Rest Mg.

2. Stent nach Anspruch 1, wobei die Magnesiumlegierung im Wesentlichen aus Folgendem besteht: einem Seltenerdmetallgehalt von bis zu 10 Gew.-%, wobei der Seltenerdmetallgehalt aus Folgendem besteht:
0,25-9 Gew.-% Sc; und
0,25-5 Gew.-% Y;
einem von:
1-3,5 Gew.-% Li;
0,5-1 Gew.-% Mn;
Rest Mg.

3. Stent nach Anspruch 1, wobei die Magnesiumlegierung im Wesentlichen aus Folgendem besteht: einem Seltenerdmetallgehalt von bis zu 10 Gew.-%, wobei der Seltenerdmetallgehalt aus Folgendem besteht:
0,25-9 Gew.-% Sc; und
0,25-5 Gew.-% Y;
1-3,5 Gew.-% Li; und
Rest Mg.

4. Stent nach Anspruch 1, wobei die Magnesiumlegierung im Wesentlichen aus Folgendem besteht:
1 Gew.-% Sc;
0,5 Gew.-% Y;
1 Gew.-% Li; und
Rest Mg.

## Revendications

1. Endoprothèse formée à partir d'un alliage de magnésium, l'alliage de magnésium étant constitué essentiellement de :
une teneur en terres rares allant jusqu'à 10 % en poids, la teneur en terres rares étant constituée de :
0,25-9 % en poids de Sc ; et
0,25-5 % en poids de Y ;
l'un parmi :
1-3,5 % en poids de Li ;
0,5-1 % en poids de Mn ;
0,3-1 % en poids de Zr ;
solde Mg.

2. Endoprothèse selon la revendication 1, l'alliage de magnésium étant constitué essentiellement de : une teneur en terres rares allant jusqu'à 10 % en poids, la teneur en terres rares étant constituée de :
0,25-9 % en poids de Sc ;
et 0,25-5 % en poids de Y ;
l'un parmi :
1-3,5 % en poids de Li ;
0,5-1 % en poids de Mn ;
solde Mg.

3. Endoprothèse selon la revendication 1, l'alliage de magnésium étant constitué essentiellement de : une teneur en terres rares allant jusqu'à 10 % en poids, la teneur en terres rares étant constituée de :
0,25-9 % en poids de Sc ;
et 0,25-5 % en poids de Y ;
1-3,5 % en poids de Li ; et
solde Mg.

4. Endoprothèse selon la revendication 1, dans laquelle l'alliage de magnésium est constitué essentiellement de :
1 % en poids de Sc ;
0,5 % en poids de Y ;
1 % en poids de Li ; et
solde Mg.
